(19) Europäisches Patentamt / European Patent Office / Office européen des brevets

(11) **EP 2 558 954 B1**

(12) **FASCICULE DE BREVET EUROPEEN**

(45) Date de publication et mention
de la délivrance du brevet:
**02.10.2019 Bulletin 2019/40**

(51) Int Cl.:
***G06F 17/14*** *(2006.01)*    ***A61B 5/0452*** *(2006.01)*

(21) Numéro de dépôt: **11720145.9**

(22) Date de dépôt: **12.04.2011**

(86) Numéro de dépôt international:
**PCT/FR2011/050828**

(87) Numéro de publication internationale:
**WO 2011/128571 (20.10.2011 Gazette 2011/42)**

(54) **PROCÉDÉ ET SYSTÈME D'ANALYSE DE L'ACTIVITÉ CARDIAQUE D'UN PATIENT ET APPLICATIONS CORRESPONDANTES**

VERFAHREN UND SYSTEM ZUR ANALYSE DER HERZAKTIVITÄT EINES PATIENTEN UND VERWENDUNG

METHOD AND SYSTEM FOR ANALYSING THE CARDIAC ACTIVITY OF A PATIENT AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.04.2010 FR 1052788**

(43) Date de publication de la demande:
**20.02.2013 Bulletin 2013/08**

(73) Titulaire: **Centre National de la Recherche Scientifique (C.N.R.S.) 75016 Paris (FR)**

(72) Inventeur: **HANUSSE, Patrick F-33600 Pessac (FR)**

(74) Mandataire: **Lavoix 2, place d'Estienne d'Orves 75441 Paris Cedex 09 (FR)**

(56) Documents cités:
**FR-A1- 2 955 187**

• **Centre de Recherche Paul Pascal: "Présentation de l'équipe Structures et dynamiques non-linéaires", Site Internet du Centre de Recherche Paul Pascal, Bordeaux, France, 9 octobre 2005 (2005-10-09), XP007917496, Extrait de l'Internet: URL:http://www.crpp.bordeaux.cnrs.fr [extrait le 2010-09-28]**

• **Centre de Recherche Paul Pascal: "Archive du séminaire du Centre de Recherche Paul Pascal", Site Internet du Centre de Recherche Paul Pascal, Bordeaux, France, XP007917497, Extrait de l'Internet: URL:http://www.crpp-bordeaux.cnrs.fr [extrait le 2011-03-07]**

• **Hanusse P: "Théorie de l'anharmonicité des phénomènes périodiques non-linéaires", Résumé d'un exposé soumis pour la 13e Rencontre du Non-Linéaire, 10-12 mars 2010, Paris, France, 8 janvier 2010 (2010-01-08), XP055012353, Serveur du site Internet de la 13e Rencontre du Non Linéaire Extrait de l'Internet: URL:http://nonlineaire.univ-lille1.fr/SNL/media/2010/resumes/hanusse_patrick.pdf [extrait le 2011-11-17]**

• **Institut Henri Poincaré: "Résumés des exposés de la 13e Rencontre du Non-Linéaire (version du 13.01.2010)", Serveur du site Internet de la 13e Rencontre du Non Linéaire, 10-12 mars 2010, Paris, France, 13 janvier 2010 (2010-01-13), XP055012351, Extrait de l'Internet: URL:http://nonlineaire.univ-lille1.fr/SNL/media/2010/resumes/ResumesRNL2010sel.pdf [extrait le 2011-11-17]**

- Institut Henri Poincaré: "Résumés des exposés de la 13e Rencontre du Non-Linéaire (version du 08.03.2010)", Serveur du site Internet de la 13e Rencontre du Non Linéaire, 10-12 mars 2010, Paris, France, 8 mars 2010 (2010-03-08), XP055012352, Extrait de l'Internet: URL:http://nonlineaire.univ-lille1.fr/SNL/media/2010/resumes/ResumesRNL2010.pdf [extrait le 2011-11-17]
- Science Non-Linéaire: "Index of /SNL/media/2010/resumes", Serveur du site Internet de la 13e Rencontre du Non Linéaire, 10-12 mars 2010, Paris, France, XP055012387, Extrait de l'Internet: URL:http://nonlineaire.univ-lille1.fr/SNL/media/2010/resumes/ [extrait le 2011-11-17]
- Science Non-Linéaire: "Programme de la 13e Rencontre du Non-Linéaire", Serveur du site Internet de la 13e Rencontre du Non Linéaire, 10-12 mars 2010, Paris, France, 8 mars 2010 (2010-03-08), XP055012355, Extrait de l'Internet: URL:http://nonlineaire.univ-lille1.fr/SNL/media/2010/programme/ProgrammeRNL2010.pdf [extrait le 2011-11-17]
- Kraskov A et al: "Extracting phases from aperiodic signals", arXiv:cond-mat/0409382v1 [cond-mat.dis-nn], 15 septembre 2004 (2004-09-15), XP002627501, Extrait de l'Internet: URL:http://arxiv.org/PS_cache/cond-mat/pdf/0409/0409382v1.pdf [extrait le 2011-03-07]
- HANUSSE P: "A novel approach to anharmonicity for a wealth of applications in nonlinear science technologies", INTERNATIONAL CONFERENCE ON APPLICATIONS IN NONLINEAR DYNAMICS (ICAND 2010), 21-24 SEPTEMBER 2010, ALBERTA, CANADA, AIP CONFERENCE PROCEEDINGS, vol. 1339, 19 avril 2011 (2011-04-19), pages 303-308, XP055012260,
- Patrick Hanusse: "Théorie de l'anharmonicité des phénomènes périodiques non-linéaires", Rencontre du non-linéaire 2012, 15-16 March 2012, March 2012 (2012-03), pages 127-132, XP055205701, Retrieved from the Internet: URL:http://nonlineaire.univ-lille1.fr/SNL/media/2012/CR/Hanusse.pdf [retrieved on 2015-07-31]
- MCSHARRY ET AL.: "A dynamical model for generating electrocardiogram signals", IEEE TRANSACTIONS ON BIOMEDICAL ENGINERRING, vol. 50, no. 3, March 2003 (2003-03), pages 289-294,

**Description**

La présente invention concerne un procédé d'analyse de l'activité cardiaque d'un patient, comprenant les étapes d'acquisition d'au moins un signal électrique cardiaque comprenant au moins un signal élémentaire correspondant à un battement cardiaque, d'extraction, à partir dudit signal élémentaire, d'au moins une onde élémentaire dont la forme générale peut être exprimée par $x(t) = x_0 + x_1 \cos(\Phi(t))$, où $\Phi(t)$ est la phase de ladite onde élémentaire, et d'analyse de ladite onde élémentaire, et un système d'analyse correspondant.

Elle concerne également des applications de ceux-ci à un stimulateur et à un défibrillateur cardiaque.

Elle s'applique en particulier au domaine de l'électrocardiographie et à l'analyse d'électrocardiogrammes.

L'électrocardiogramme est la représentation graphique de l'activité du coeur, enregistrées par des électrodes placées à la surface du corps. En électrocardiographie conventionnelle, l'activité électrique du coeur est étudiée par l'enregistrement d'une série de 12 dérivations, chaque dérivation correspondant à une ligne de tension réunissant deux électrodes placées en deux points déterminés de la surface du corps et entre lesquelles sont enregistrées des différences de potentiel. Les courbes ainsi enregistrées représentent les courants de dépolarisation et de repolarisation des muscles auriculaires et ventriculaires, qui se répètent de manière quasi-périodique à chaque cycle cardiaque.

Chacune des dérivations correspond à un signal, appelé par la suite signal ECG, comprenant une succession de signaux élémentaires, ou complexe PQRST, représentant chacun un cycle cardiaque complet, se répétant à intervalles de temps réguliers.

Un tel signal élémentaire est composé d'une succession d'ondes élémentaires, positives ou négatives, de part et d'autre d'une ligne dite « isoélectrique » correspondant au repos cardiaque. Ces ondes positives ou négatives résultent de processus physiologiques bien définis, et sont généralement identifiées par les labels standardisés P, Q, R, S et T. L'onde P est générée lors de la dépolarisation de l'oreillette, le complexe QRS représente la dépolarisation du ventricule et l'onde T est générée lors de la repolarisation de ce ventricule.

L'analyse des signaux élémentaires et de leur variabilité permet de détecter d'éventuelles anomalies cardiaques. Cette analyse, longtemps réalisée par la seule lecture du tracé papier du signal, comprend la mesure du rythme cardiaque, généralement déterminé à partir de l'intervalle entre deux ondes R, la mesure des amplitudes et durées ainsi que l'examen de la morphologie de l'onde P, du complexe QRS, de l'onde T, de l'intervalle PR, du segment ST, et de l'intervalle QT.

Des techniques de traitement du signal permettent désormais de réaliser une analyse automatique du signal ECG, et fournissent des résultats synthétiques à partir desquels le médecin peut établir un diagnostic. Cette analyse automatique est généralement effectuée par décomposition du signal ECG en signaux élémentaires, comprenant chacun un complexe PQRST, décomposition de chacun de ces signaux élémentaires en ondes élémentaires (ondes P, Q, R, S et T), puis analyse de chaque onde élémentaire et caractérisation de ces ondes par plusieurs paramètres. Une synthèse de ces paramètres et de leur évolution temporelle est enfin réalisée, et permet de détecter d'éventuelles anomalies.

De nombreuses méthodes d'analyse et de caractérisation d'un signal ECG ont été proposées. En particulier, l'analyse fréquentielle du signal permet de décrire ce signal dans l'espace de Fourier. La décomposition de Fourier consiste en effet à décomposer un signal périodique de fréquence f en une somme infinie de fonctions sinusoïdales de fréquences multiples de f, pondérées par les coefficients de Fourier. Ces coefficients de Fourier, qui constituent un codage du signal analysé, sont des paramètres caractéristiques de ce signal. En pratique, le nombre de coefficients de Fourier conservés est limité, et seuls les premiers termes de la décomposition de Fourier sont gardés. Ces termes doivent cependant être en nombre suffisant pour que le signal synthétisé à partir du codage soit aussi proche que possible du signal d'origine.

Or, le signal ECG est un signal fortement anharmonique, c'est-à-dire non-linéaire, et la décomposition de Fourier de ce signal nécessite de conserver un grand nombre de coefficients, coefficients auxquels il est difficile de donner un sens physique. De plus, cette décomposition, si elle permet de décrire la répartition des composantes fréquentielles du signal, ne renseigne nullement sur les instants de l'apparition de celles-ci, et ne permet pas de caractériser les différentes ondes (onde P, complexe QRS, onde T...) du signal et leur forme.

Cet inconvénient peut être pallié par une modélisation du signal ECG obtenue par décomposition de ce signal en une somme d'ondelettes ou de gaussiennes. Cependant, cette méthode nécessite également la détermination d'un très grand nombre de paramètres pour que la modélisation soit de qualité suffisante. Par ailleurs, les ondes P et T, difficilement assimilables à des gaussiennes, sont généralement mal modélisées.

On connaît par ailleurs du document « A dynamical model for generating electrocardiogram signals » (McSharry et al., IEEE Transactions on Biomedical Enginerring 50(3) : 289-294, Mars 2003), une méthode de modélisation de signaux ECG permettant de générer des signaux ECG à partir de paramètres statistiques tels que la moyenne et l'écart-type du rythme cardiaque, et de paramètres de morphologie relatifs notamment à la morphologie du complexe PQRST.

Cependant, cette méthode ne permet pas d'analyser directement un signal ECG. De plus, elle repose sur des calculs complexes, nécessitant d'effectuer une intégration numérique complète pour chaque jeu de paramètres fixé.

L'invention a donc pour but de permettre l'analyse des formes d'onde de signaux d'activité cardiaque, au moyen d'un petit nombre de paramètres, par rapport au nombre de paramètres nécessaires à l'analyse par série de Fourier ou à la décomposition en ondelettes ou en gaussiennes, lesdits paramètres étant porteurs d'un sens physique et constituant une signature simple et explicite de la forme de ces signaux.

A cet effet, l'invention a pour objet un procédé d'analyse de l'activité cardiaque d'un patient, un système d'analyse de l'activité cardiaque d'un patient, un stimulateur cardiaque et un défibrillateur cardiaque selon les revendications annexées.

Ainsi réalisé, le procédé selon l'invention permet d'analyser les signaux ECG et de caractériser ces signaux au moyen d'un petit nombre de paramètres, par rapport aux procédés de décomposition selon l'état de la technique. De plus, ces paramètres ont un sens physique, et sont caractéristiques des formes d'onde des complexes PQRST.

L'invention sera mieux comprise au regard d'un exemple de réalisation de l'invention qui va maintenant être décrit en faisant référence aux figures annexées parmi lesquelles :

- la figure 1 illustre un complexe PQRST d'un signal ECG ;
- la figure 2 représente de manière schématique le système selon un mode de réalisation de l'invention ; et
- la figure 3 est un schéma synoptique illustrant le procédé selon un mode de réalisation de l'invention.

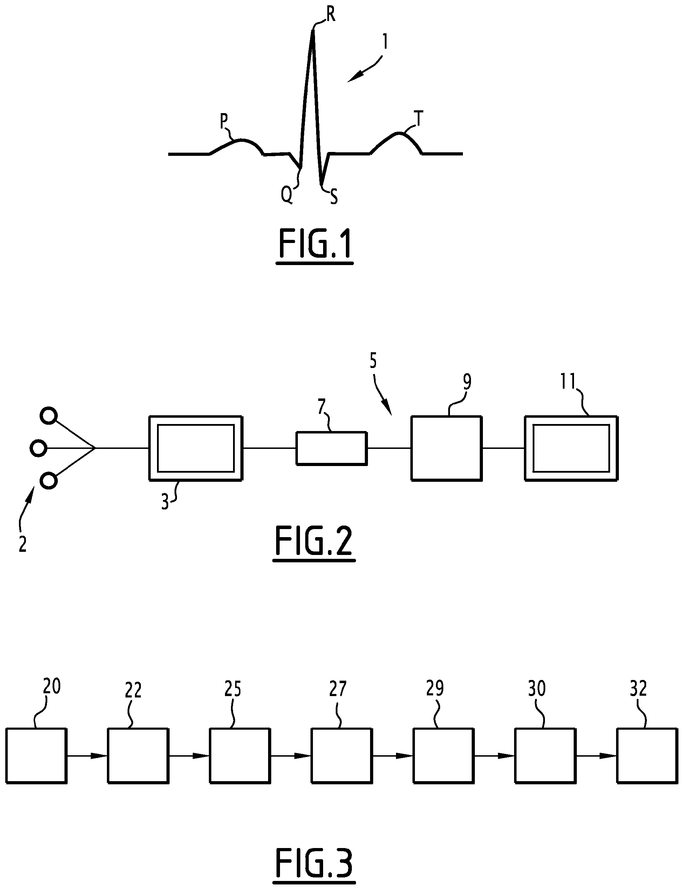

On a représenté sur la figure 1 un tracé illustrant la forme d'un signal élémentaire 1 d'un signal ECG, comprenant un complexe PRQST. Sur ce tracé, le temps est représenté en abscisse, et la tension en ordonnée. On reconnaît sur ce tracé l'onde P, le complexe QRS, et l'onde T, générés lors d'un battement cardiaque.

On a représenté sur la figure 2 un système d'acquisition et d'analyse de signaux cardiaques.

Ce système comporte des moyens d'acquisition de signaux ECG, comprenant une pluralité d'électrodes 2 de mesure, placées en différents endroits du corps d'un patient, et reliées à un moniteur électrocardiographique 3 (appelé également moniteur ECG).

Ce système comporte également des moyens 5 de traitement et d'analyse de signaux ECG, comprenant un convertisseur analogique/numérique 7 et des moyens 9 d'analyse de signaux numériques, par exemple un processeur. L'entrée du convertisseur 7 est reliée à une sortie du moniteur ECG 3, et l'entrée du processeur 9 est reliée à la sortie du convertisseur 7.

Le système comporte par ailleurs un moniteur 11, relié à une sortie du processeur 9.

Les électrodes 2 de mesure sont aptes à recevoir, lorsqu'elles sont placées sur le corps du patient, les signaux électriques générés dans le coeur, dont l'amplitude est de l'ordre du millivolt. L'ensemble des électrodes permet de recevoir les signaux des multiples dérivations, généralement au nombre de six ou douze.

Le moniteur ECG 3 est apte à afficher, au fur et à mesure de leur acquisition par les électrodes 1, les signaux ECG, sous la forme de courbes représentant en abscisse le temps, par exemple avec une échelle de 25 mm/s, et on ordonnée la tension, par exemple avec une échelle de 1 cm/mV. Ces courbes sont quasi-périodiques, chaque période correspondant à un battement cardiaque, et leur forme est variable suivant la dérivation considérée. La figure 1 représente ainsi la forme du signal d'une de ces dérivations, sur une période correspondant à un battement cardiaque.

Le moniteur 3 est également apte à délivrer en sortie des signaux analogiques, correspondant aux signaux ECG perçus par les électrodes.

Le convertisseur analogique/numérique 7 est apte à numériser un signal ECG analogique, par échantillonnage de ce signal avec une fréquence d'échantillonnage prédéterminée, par exemple 256 Hz.

Le processeur 9 est apte à filtrer un signal ECG numérique, à analyser ce signal de manière à en extraire des paramètres caractéristiques de la forme de ce signal et du rythme cardiaque, et à réaliser une synthèse de ces paramètres.

La figure 3 est un schéma synoptique illustrant l'acquisition et l'analyse d'un signal d'activité cardiaque au moyen du système décrit en référence à la figure 2, selon un mode de réalisation de l'invention.

Dans une première étape 20 d'acquisition, les signaux électriques générés par l'activité cardiaque sont perçus par les électrodes 2, et transmis au moniteur ECG 3, sous la forme de signaux analogiques. Le moniteur ECG 3 affiche, au fur et à mesure de leur réception, plusieurs courbes représentant ces signaux. Cet affichage permet notamment à un praticien de s'assurer de la bonne acquisition des signaux.

Les signaux perçus par les électrodes 2 sont au nombre de douze par exemple, chacun de ces signaux correspondant à une dérivation particulière. Le procédé selon l'invention permet d'analyser chacune de ces dérivations, mais on ne détaillera dans la suite de la description que l'analyse d'une seule de ces dérivations. Ainsi, dans la suite de la description, on désignera par « signal ECG » un signal associé à l'une de ces dérivations.

Dans une étape 22 de numérisation, le moniteur ECG 3 transmet le signal ECG analogique au convertisseur analogique/numérique 7 qui numérise ce signal, par échantillonnage et quantification.

Le signal ECG numérique obtenu à l'issue de l'étape 22 est transmis au processeur 9, qui analyse ce signal afin d'en extraire des paramètres caractéristiques.

Ce signal est composé d'une succession de signaux élémentaires 1, correspondant chacun à un battement cardiaque, et formant un complexe PQRST. Il n'est cependant pas rigoureusement périodique, en raison notamment de la variabilité du rythme cardiaque et de la forme du complexe QRS. Par ailleurs, ce signal n'est pas dû à la seule activité électrique du coeur. En effet, l'activité des muscles autres que cardiaques, notamment les muscles respiratoires, et les interférences d'appareils électriques, par exemple les moniteurs 3 et 11, génèrent des signaux parasites de hautes et de basses fréquences qui sont également reçus par les électrodes 2.

Ainsi, dans une étape 25, un filtrage numérique est appliqué au signal ECG numérique, de manière à éliminer ces signaux parasites.

Dans une étape 27, le signal ECG filtré est décomposé en signaux élémentaires Y(t), comprenant chacun un complexe PQRST, au moyen de méthodes connues, par exemple par détection de l'onde R, qui est généralement l'onde la plus fine et la plus ample du complexe PQRST. Lors de cette décomposition, le rythme cardiaque et sa variabilité sont déterminés, par calcul des différents intervalles de temps séparant les ondes P successives du signal.

Puis, le processeur 9 analyse chacun des signaux élémentaires Y(t), et en particulier la morphologie des complexes PQRST.

A cette fin, chaque signal élémentaire est analysé et décomposé dans une étape 29 en une somme d'ondes élémentaires correspondant chacune à une onde P, Q, R, S ou T du signal élémentaire Y(t).

Chaque signal élémentaire Y(t) est ainsi décrit par :

$$Y(t) = x_P(t - t_p) + x_Q(t - t_Q) + x_R(t - t_R) + x_S(t - t_S) + x_T(t - t_T)$$

où $x_P$, $x_Q$, $x_R$, $x_S$ et $x_T$ désignent respectivement les ondes P, Q, R, S et T, et $t_P$, $t_Q$, $t_R$, $t_S$ et $t_T$ désignent les origines temporelles de ces ondes, c'est-à-dire les instants auxquels ces ondes apparaissent dans le signal élémentaire.

Chacune des ondes élémentaires est alors analysée dans une étape 30, et caractérisée par un petit nombre de paramètres. L'analyse de chacune de ces ondes est réalisée selon les mêmes étapes. Ainsi, on désignera par x(t), dans la suite de la description, une onde élémentaire, quel que soit son type, et on supposera que son origine temporelle est à l'instant t=0.

Chaque onde élémentaire $x(t)$ est un signal anharmonique, qui peut être décrit sous la forme suivante :

$$x(t) = x_0 + x_1 \cos(\Phi(t)) \qquad (1)$$

dans laquelle toute la dépendance temporelle est contenue dans la fonction de phase $\Phi$.

Cette onde élémentaire $x(t)$ est considérée comme un signal périodique de période T, T étant la période du rythme cardiaque mesurée localement. Dans la suite de la description, on considèrera une période normalisée de valeur $2\pi$.

Dans un signal anharmonique, la principale contribution à l'anharmonicité provient de la brisure de symétrie de la dynamique de phase. Ainsi, toute l'information dynamique pertinente est exprimée par la dynamique de phase. Lors de l'analyse de l'onde x(t), il convient donc d'étudier cette phase $\Phi(t)$, et en particulier la dynamique de phase exprimée par la fonction F, dérivée de la fonction $\Phi$ par rapport au temps t :

$$F(\Phi) = \frac{d\Phi}{dt} \qquad (2)$$

Ainsi, la morphologie de l'onde x(t) est complètement déterminée par la connaissance de F.

Lors de l'étape d'analyse 30 du procédé selon l'invention, il convient donc de décrire cette fonction F au moyen d'un très petit nombre de paramètres. On entendra par petit nombre de paramètres un nombre de paramètres réduit par rapport au nombre de paramètres nécessaire à la décomposition de la même fonction, au moyen des séries de Fourier, avec un niveau de précision équivalent.

Cette étape d'analyse 30 comprend ainsi une première étape consistant à exprimer la phase $\Phi$, et en particulier la fonction F, dérivée de $\Phi$ par rapport au temps.

Dans le cas le plus simple, et pour une onde de période $2\pi$, la dynamique de phase peut être écrite sous la forme :

$$F(\Phi) = \frac{d\Phi}{dt} = \frac{1 + r^2 + 2r\cos(\Phi)}{1 - r^2} \qquad (3)$$

appelée équation de phase.

La fonction F présente dans ce cas une symétrie de réflexion par rapport à l'axe $\Phi=0$. Cette expression de la dynamique de phase ne contient qu'un seul paramètre, r, qui varie dans l'intervalle [0,1]. La limite r=0 correspond à une onde harmonique, la limite r=1 à une onde infiniment anharmonique.

Dans un exemple non couvert par les revendication, l'onde x(t), qui peut être écrite sous la forme :

$$x(t) = x_0 + x_1 \cos\big(\Phi(t,r) - \Phi_0\big) \qquad (4)$$

où $\Phi_0$ est une origine de phase, est décomposée et réécrite sous une forme faisant intervenir les paramètres r et $\Phi_0$ :

$$x(t) = x_0 + a_1 h\sin(t,r) + b_1 h\cos(t,r) \qquad (5)$$

avec $a_1 = x_1 \cos(\Phi_0)$ et $b_1 = -x_1 \sin(\Phi_0)$, et dans laquelle on a défini les fonctions hcos et hsin suivantes :

$$h\cos : (t,r) \rightarrow \frac{\big(1+r^2\big)\cos(t) + 2r}{1+r^2 - 2r\cos(t)} \qquad (6)$$

$$h\sin : (t,r) \rightarrow \frac{\big(1-r^2\big)\sin(t)}{1+r^2 - 2r\cos(t)} \qquad (7)$$

Ainsi, la décomposition de l'onde x(t) fait intervenir seulement deux paramètres, r et $\Phi_0$.

r, appelé paramètre d'anharmonicité, mesure le degré d'anharmonicité de l'onde, la limite r=0 correspond à une onde harmonique, la limite r=1 à une onde infiniment anharmonique. Par ailleurs, le paramètre $\Phi_0$, qui définit la composition de l'onde dans les deux fonctions hcos et hsin, est un paramètre de morphologie, qui correspond à l'angle de symétrie de réflexion de la dynamique de phase.

Selon l'invention, dans le cas général, c'est-à-dire pour une onde périodique quelconque, l'équation de phase peut s'écrire sous la forme :

$$F(\Phi) = \frac{P_n(\Phi)}{Q_m(\Phi)} \qquad (8)$$

dans laquelle $P_n$ et $Q_m$ sont des polynômes trigonométriques de degrés respectifs n et m. La forme générale d'un polynôme trigonométrique de degré n est :

$$P_n(\Phi) = a_0 + \sum_{k=1}^{n} a_k \cos(k\Phi) + b_k \sin(k\Phi) \qquad (9)$$

L'analyse de l'onde x(t) comprend donc la détermination d'une expression de $\Phi$ faisant intervenir un petit nombre de paramètres, ce qui permet de déterminer une expression de l'onde x(t) en fonction de ces paramètres.

Avantageusement, l'équation de phase (2) peut être réécrite sous la forme :

$$\frac{1}{F(\Phi)} = \frac{dt}{d\Phi} = \frac{Q_m(\Phi)}{P_n(\Phi)} \qquad (10)$$

La factorisation du polynôme $P_n(\Phi)$ permet de transformer $\dfrac{1}{F(\Phi)}$ en une somme de termes simples, ce qui permet de réécrire l'équation de phase sous la forme :

$$\frac{dt}{d\Phi} = a_0 + \sum_{k=1}^{n} \frac{a_k \cos(\Phi - p_k) + b_k \sin(\Phi + p_k)}{\left(1 + r_k^2 - 2r_k \cos(\Phi + p_k)\right)} \tag{11}$$

dans laquelle les paramètres $r_k$, compris entre 0 et 1, mesurent l'anharmonicité de l'onde x(t), et les paramètres $p_k$ caractérisent sa morphologie.

La période T de l'onde peut être déterminée en intégrant cette équation par rapport à $\Phi$, entre 0 et $2\pi$ :

$$T = \int_{\Phi=0}^{\Phi=2\pi} \frac{d\Phi}{F(\Phi)} = 2\pi\left(a_0 + \sum_{k} \frac{r_k a_k}{1 - r_k^2}\right) \tag{12}$$

A partir de ce résultat, et des contraintes selon lesquelles la période est égale à $2\pi$ et l'onde est harmonique lorsque les coefficients $r_k$ sont tous nuls, l'équation de phase peut être exprimée ainsi :

$$\frac{dt}{d\Phi} = 1 + \sum_{k=1}^{n} D_k(\Phi - p_k) \tag{13}$$

Où la fonction $D_k$ est définie par :

$$D_k : \Phi \rightarrow \frac{r_k\left(a_k \cos(\Phi) + b_k \sin(\Phi) - a_k\right)}{\left(1 + r_k^2 - 2r_k \cos(\Phi)\right)} \tag{14}$$

Et vérifie :

$$\int_{\Phi=0}^{\Phi=2\pi} D_k(\Phi)d\Phi = 0 \tag{15}$$

La définition des fonctions des fonctions polycos et polysin, notées $pcos_n$ et $psin_n$, qui s'expriment par :

$$p\cos_n(t,r) = \sum_{k=1}^{\infty} \cos(kt)\frac{r^k}{k^n} \tag{16}$$

$$p\sin_n(t,r) = \sum_{k=1}^{\infty} \sin(kt)\frac{r^k}{k^n} \tag{17}$$

et possèdent entre autre les propriétés suivantes :

$$p\cos_0(t,r) = \frac{r(\cos(t) - r)}{1 + r^2 - 2r\cos(t)} \tag{18}$$

$$p\sin_0(t,r) = \frac{r\sin(t)}{1 + r^2 - 2r\cos(t)} \tag{19}$$

$$p\cos_1(t,r) = -\frac{1}{2}\ln\left(1 + r^2 - 2r\cos(t)\right) \tag{20}$$

$$p\sin_1(t,r) = \tan^{-1}\left(\frac{r\sin(t)}{1 - r\cos(t)}\right) \tag{21}$$

permet de réécrire l'équation de phase sous la forme :

$$\frac{dt}{d\Phi} = 1 + \sum_{k=1}^{n} a_k\, p\cos_0\left(\Phi - p_k, r_k\right) + b_k\, p\sin_0\left(\Phi - p_k, r_k\right) \tag{22}$$

La résolution de cette équation permet d'accéder à une expression analytique de $t(\Phi)$, qui s'exprime par :

$$t(\Phi) = \Phi + \sum_{k=1}^{n} a_k\, p\sin_1\left(\Phi - p_k, r_k\right) - b_k\, p\cos_1\left(\Phi - p_k, r_k\right) \tag{23}$$

Le temps t est donc exprimé en fonction de la phase $\Phi$, et de manière duale la phase $\Phi$ est exprimée en fonction du temps t, à l'aide de paramètres indépendants clairement définis, qui mesurent l'anharmonicité (paramètres r ou $r_k$), et la morphologie (paramètres $\Phi_0$ ou $p_k$).

Ainsi, lors de l'étape 30 d'analyse, le processeur 9 code chaque onde élémentaire x(t) au moyen d'un petit nombre de paramètres. Selon un mode de réalisation, chaque onde élémentaire x(t) est décrit de manière encore plus précise par deux couples de paramètres $(r_1, p_1)$ et $(r_2, p_2)$, complétés de leurs poids respectifs.

Chacune des ondes élémentaires, donc chacun des complexes PQRST du signal ECG, est donc caractérisée par un nombre restreint de paramètres, porteurs d'un sens physique car représentatifs de la non-linéarité et de la morphologie de ces complexes.

Dans une étape 32, le processeur 9 synthétise les paramètres du signal ECG déterminés lors des étapes 27 et 29, c'est-à-dire le rythme cardiaque et les paramètres d'harmonicité et de morphologie, en déterminant notamment la moyenne et l'écart-type de chacun de ces paramètres sur l'ensemble du signal ECG. Ces valeurs sont affichées sur le moniteur 11, et peuvent servir de base à un diagnostic, par un praticien ou automatique, par comparaison des ces valeurs avec des valeurs tabulées correspondant à des anomalies cardiaques particulières.

Le procédé selon l'invention permet ainsi d'analyser l'activité cardiaque, et d'extraire des signaux électriques générés par l'activité cardiaque un nombre restreint de paramètres, permettant une représentation compacte et pertinente de la forme d'onde de ces signaux.

Il devra toutefois être compris que l'exemple de réalisation présenté ci-dessus n'est pas limitatif.

Notamment, selon un autre mode de réalisation, le signal d'activité cardiaque est un électrogramme, recueilli par des électrodes placées sur des sondes endocavitaires.

Le système et le procédé selon l'invention peuvent ainsi être mis en oeuvre dans un stimulateur ou un défibrillateur implanté, l'analyse continue des signaux d'activité cardiaque selon l'invention permettant une détection automatique de potentielles anomalies, et le déclenchement d'une stimulation du coeur.

Bien entendu, d'autres modes de réalisation peuvent être envisagés.

**Revendications**

1. Système d'analyse de l'activité cardiaque d'un patient comprenant des moyens (2) pour acquérir au moins un signal électrique cardiaque comprenant au moins un signal élémentaire (1) correspondant à un battement cardiaque, et des moyens pour extraire à partir dudit signal élémentaire (1) au moins une onde élémentaire P, Q, R, S ou T, l'onde élémentaire étant de la forme $x(t) = x_0 + x_1 \cos(\Phi(t))$, où $\Phi(t)$ est la phase de ladite onde élémentaire (P, Q, R, S, T), **caractérisé en ce que** ledit système comporte :

   - des moyens (9) pour analyser ladite onde élémentaire P, Q, R, S ou T afin de caractériser par un nombre restreint de paramètres ladite onde élémentaire, en exprimant le temps t en fonction de la phase $\Phi$ de ladite onde élémentaire selon l'expression analytique suivante :

$$t(\Phi) = \Phi + \sum_{k=1}^{n} a_k \, p\sin_1(\Phi - p_k, r_k) - b_k \, p\cos_1(\Phi - p_k, r_k),$$

expression dans laquelle :

$$p\cos_1(u, v) = -\frac{1}{2}\ln\left(1 + v^2 - 2v\cos(u)\right),$$

$$p\sin_1(u, v) = \tan^{-1}\left(\frac{v\sin(u)}{1 - v\cos(u)}\right),$$

$a_k$ et $b_k$ sont des poids, les paramètres $r_k$, compris entre 0 et 1, mesurent l'anharmonicité de ladite onde élémentaire, et les paramètres $p_k$ caractérisent la morphologie de ladite onde élémentaire ; et,
- des moyens pour comparer les paramètres déterminés pour ladite onde élémentaire en sortie des moyens pour analyser, à des valeurs tabulées de ces paramètres préalablement enregistrées, de manière à détecter des anomalies cardiaques particulières.

2. Système selon la revendication 1, dans lequel les moyens pour analyser décrivent ladite onde élémentaire par deux couples de paramètres $r_1$, $p_1$ et $r_2$, $p_2$, complétés de leurs poids respectifs.

3. Stimulateur cardiaque comprenant un système d'analyse de l'activité cardiaque selon l'une des revendications 1 à 2.

4. Défibrillateur cardiaque comprenant un système d'analyse de l'activité cardiaque selon l'une des revendications 1 à 2.

**Patentansprüche**

1. System zur Analyse der Herztätigkeit eines Patienten, das Mittel (2) zum Beschaffen mindestens eines elektrischen Herzsignals, das mindestens ein Grundsignal (1) entsprechend einem Herzschlag aufweist, und Mittel zum Extrahieren von mindestens einer Grundwelle P, Q, R, S oder T aus dem Grundsignal (1) umfasst, wobei die Grundwelle die Form $x(t) = x_0 + x_1 \cos(\Phi(t))$ hat, wobei $\Phi(t)$ die Phase der Grundwelle (P, Q, R, S, T) ist, **dadurch gekennzeichnet, dass** das System aufweist:

- Mittel (9) zum Analysieren der Grundwelle P, Q, R, S oder T, um durch eine begrenzte Anzahl von Parametern die Grundwelle zu charakterisieren, indem die Zeit t abhängig von der Phase $\Phi$ der Grundwelle gemäß dem folgenden analytischen Ausdruck ausgedrückt wird:

$$t(\Phi) = \Phi + \sum_{k=1}^{n} a_k \, p\sin_1(\Phi - p_k, r_k) - b_k \, p\cos_1(\Phi - p_k, r_k)$$

in dem:,

$$p\cos_1(u, v) = -\frac{1}{2}\ln(1 + v^2 - 2v\cos(u)),$$

$$p\sin_1(u, v) = \tan^{-1}\left(\frac{v\sin(u)}{1 - v\cos(u)}\right),$$

und $a_k$ und $b_k$ Gewichte sind, die Parameter $r_k$ zwischen 0 und 1 die Anharmonizität der Grundwelle messen und die Parameter $p_k$ die Morphologie der Grundwelle charakterisieren; und

- Mittel zum Vergleichen der für die Grundwelle bestimmten Parameter am Ausgang der Analysemittel mit vorher gespeicherten Tabellenwerten dieser Parameter, um besondere Herzanomalien zu detektieren.

2. System nach Anspruch 1, bei dem die Analysemittel die Grundwelle durch zwei Parameterpaare $r_1$, $p_1$ und $r_2$, $p_2$, die durch ihre jeweiligen Gewichte vervollständigt sind, beschreiben.

3. Herzstimulator, der ein System zur Analyse der Herzaktivität nach einem der Ansprüche 1 bis 2 umfasst.

4. Herzdefibrillator, der ein System zur Analyse der Herzaktivität nach einem der Ansprüche 1 bis 2 umfasst.

**Claims**

1. System for analysing the cardiac activity of a patient comprising means (2) for acquiring at least one cardiac electric signal comprising at least one elementary signal (1) corresponding to a heart beat, and means for extracting from said elementary signal (1) at least one elementary wave P, Q, R, S or T, the elementary wave being of the form $x(t) = x_0 + x_1 \cos(\Phi(t))$, wherein $\Phi(t)$ is the phase of said elementary wave (P, Q, R, S, T), **characterised in that** said system comprises :

   - means (9) for analysing said elementary wave P, Q, R, S or T in order to characterise said elementary wave with a reduced number of parameters, by expressing the time t as a function of the phase $\Phi$ of said elementary wave according the following analytical expression :

$$t(\Phi) = \Phi + \sum_{k=1}^{n} a_k p\sin_1(\Phi - p_k, r_k) - b_k p\cos_1(\Phi - p_k, r_k),$$

   expression wherein :

$$p\cos_1(u, v) = -\frac{1}{2}\ln(1 + v^2 - 2v\cos(u))$$

,

$$p\sin_1(u, v) = \tan^{-1}\left(\frac{v\sin(u)}{1 - v\cos(u)}\right),$$

   $a_k$ and $b_k$ are weights, parameters $r_k$, comprised between 0 et 1, measure the anharmonicity of said elementary wave, and parameters $p_k$ characterise the morphology of said elementary wave ; and,
   - means for comparing the parameters determined for said elementary wave at the output of the means for analysing, with tabulated values of these parameters previously recorded, in order to detect particular cardiac anomalies.

2. System according to claim 1, wherein the means for analysing describe the elementary wave with two pairs of parameters $r_1$, $p_1$ and $r_2$, $p_2$, completed with their respective weight.

3. Cardiac pacemaker comprising a system for analysing the cardiac activity according to any one of claims 1 to 2.

4. Cardiac defibrillator comprising a system for analysing the cardiac activity according to any one of claims 1 to 2.

FIG.1

FIG.2

FIG.3

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MCSHARRY et al.** A dynamical model for generating electrocardiogram signais. *IEEE Transactions on Biomedical Enginerring,* Mars 2003, vol. 50 (3), 289-294